# EUROPÄISCHE PATENTANMELDUNG

(11) **EP 4 248 923 A1**
(43) Veröffentlichungstag der Anmeldung: **27.09.2023**
(21) Anmeldenummer: 22164535.1
(22) Anmeldetag: 25.03.2022
(51) Int. Cl.: A61F 9/008

(54) **SYSTEM, VORRICHTUNG UND VERFAHREN ZUR ERSTELLUNG VON PLANUNGSDATENSÄTZEN FÜR DIE GEWEBEBEHANDLUNG DER NETZHAUT EINES PATIENTEN**

(71) Anmelder: OD-OS GmbH, 14513 Teltow (DE)
(72) Erfinder: Hoff, Annemarie, 14469 Potsdam (DE); Rose, Arnd, 14532 Stahnsdorf (DE); Amthor, Kay-Uwe, 14513 Teltow (DE)
(74) Vertreter: Pfenning, Meinig & Partner mbB

(57) **Zusammenfassung**

Die Erfindung betrifft ein System zur Erstellung von Planungsdatensätzen (34, 35, 36, 37) für die Gewebebehandlung der Netzhaut (16) des Auges (4) eines Patienten mittels eines Lasers (2), mit einer Kamera (6,7), die dazu eingerichtet ist, laufend Aufnahmen der Netzhaut zu erfassen, mit einer Bildverarbeitungseinrichtung (19), die dazu eingerichtet ist, aus durch die Kamera erfassten Aufnahmen aktuelle strukturierte Bilddaten der Netzhaut zu erzeugen und bereitzustellen, mit einer Bestandsdatenerfassungseinrichtung (21,22), die dazu eingerichtet ist, Bestandsdaten eines Patienten zu erfassen und/oder bereitzustellen, und mit einer Verarbeitungseinrichtung (20), die dazu eingerichtet ist, den aktuellen strukturierten Bilddaten der Netzhaut und den Bestandsdaten des Patienten Planungsdatensätze für die Gewebebehandlung zuzuordnen, wobei in der Verarbeitungseinrichtung Vorgaben, insbesondere Muster und Regeln, für die Zuordnung von Planungsdaten zu Bilddaten und Bestandsdaten von Patienten gespeichert sind.

## Beschreibung

Die Erfindung liegt auf dem Gebiet der Medizintechnik und kann besonders vorteilhaft auf dem Gebiet der Ophthalmologie und in Verbindung mit bestimmten Ophthalmoskopen eingesetzt werden.

Für die Behandlung der Netzhaut eines Patienten werden in vielen Fällen Laserbehandlungsgeräte eingesetzt, die die Gewebebehandlung mittels eines Laserstrahls ermöglichen. Besonders vorteilhaft ist dabei die kombinierte Verwendung eines Ophthalmoskops mit einem Gerät zur Laserbehandlung, um gleichzeitig und abgestimmt eine visuelle Darstellung der Netzhaut auch während einer Behandlung zu ermöglichen.

Grundsätzlich hat ein Ophthalmoskop die Funktion, einer untersuchenden Person ein Bild von einem Auge oder Teilen eines Auges zu liefern. Insbesondere ein Ophthalmoskop, das ein Bild der Netzhaut eines Auges liefert, kann mit einem Gerät zur Laserbehandlung der Netzhaut kombiniert werden, wenn beispielsweise für die Führung des Laserstrahls wenigstens teilweise dieselbe optische Abbildungseinrichtung verwendet wird wie für die Visualisierung der Netzhaut. In diesem Fall können die Strahlengänge für die Abbildung und die Laserbehandlung beispielsweise durch Strahlteilereinrichtungen auf einem Teil ihrer Länge verzweigt werden und auf einem anderen Teil gemeinsam durch dieselben optischen Elemente verlaufen. Dadurch wird eine Beobachtung der Netzhaut gleichzeitig mit der Laserbehandlung ermöglicht, die bei getrennten Abbildungseinrichtungen für die Beobachtung und die Behandlung schon aus Platzgründen deutlich schwieriger wäre.

Aus der europäischen Patentanmeldung EP 1 875 857 A1 ist ein Ophthalmoskop mit einer Abbildungsoptik und einer Beleuchtungseinrichtung sowie einem optischen Sensor bekannt, mit dem Bilder der Netzhaut eines Auges aufgenommen werden können. Ein solches Ophthalmoskop kann auch für die Planung einer Laserbehandlung durch einen Operateur genutzt werden.

Vor dem Hintergrund des Standes der Technik liegt der vorliegenden Erfindung die Aufgabe zugrunde, die Erstellung von Behandlungsplänen für die Gewebebehandlung einer Netzhaut mittels einer Lasereinrichtung zu erleichtern und die Planungsdaten systematisch zu standardisieren und zu verbessern.

Die Aufgabe wird gemäß der Erfindung durch ein System mit den Merkmalen des Patentanspruchs 1 gelöst. Die Unteransprüche stellen vorteilhafte Implementierungen eines solchen Systems vor. Zudem bezieht sich die Erfindung zur Lösung der Aufgabe auf eine Vorrichtung und ein Verfahren zur Erstellung von Planungsdatensätzen, die samt vorteilhaften Ausführungsformen in weiteren Patentansprüchen niedergelegt sind.

Demgemäß bezieht sich die Erfindung auf ein System zur Erstellung von Planungsdatensätzen für die Gewebebehandlung der Netzhaut des Auges eines Patienten mittels eines Lasers,
- mit einer Kamera, die dazu eingerichtet ist, laufend Aufnahmen der Netzhaut zu erfassen,
- mit einer Bildverarbeitungseinrichtung, die dazu eingerichtet ist, aus durch die Kamera erfassten Aufnahmen aktuelle strukturierte Bilddaten der Netzhaut zu erzeugen und bereitzustellen,
- mit einer Bestandsdatenerfassungseinrichtung, die dazu eingerichtet ist, Bestandsdaten eines Patienten zu erfassen und/oder bereitzustellen,
und mit einer Verarbeitungseinrichtung, die dazu eingerichtet ist, den aktuellen strukturierten Bilddaten der Netzhaut und den Bestandsdaten des Patienten Planungsdatensätze für die Gewebebehandlung zuzuordnen, wobei in der Verarbeitungseinrichtung Vorgaben, insbesondere Muster und Regeln, für die Zuordnung von Planungsdaten zu Bilddaten und Bestandsdaten von Patienten gespeichert sind-Zudem kann vorteilhaft vorgesehen sein, dass das System eine Änderungseinrichtung aufweist, die auch als Korrektureinrichtung bezeichnet werden kann und die die manuelle Änderung eines Planungsdatensatzes ermöglicht. Derartige manuelle Änderungen können während oder vor allem nach der Erstellung eines Planungsdatensatzes durch einen Experten aufgrund seiner Erfahrung oder zusätzlicher Erkenntnisse vorgenommen werden.

Das erfindungsgemäße System ist dazu eingerichtet, vor Beginn der Behandlung der Netzhaut eines Patienten bei der Erstellung eines Behandlungsplans, der in der Form einer Planungsdatei beziehungsweise von Planungsdaten erstellt wird, zu unterstützen. Eine Voraussetzung dafür ist, dass das System eine Kamera oder allgemein ein bildgebendes System aufweist, die/das dazu eingerichtet ist, laufend Aufnahmen der Netzhaut zu erfassen. Eine derartige Kamera kann als optische Farb-, Infrarot- oder Schwarzweißkamera oder als scannendes System ausgebildet sein oder auch verschiedene Filtermöglichkeiten aufweisen und ermöglicht die Erfassung von aktuellen Aufnahmen der Netzhaut, bevor und/oder während Planungsdaten erzeugt werden. Dies kann beispielsweise wichtig sein, um andere erfasste oder aktuell ermittelte Daten der Netzhaut mit dem aktuell erfassten Zustand und der Positionierung der Netzhaut in dem System abgleichen zu können. Unter strukturierten grafischen oder Bilddaten sollen im Zusammenhang der vorliegenden Erfindung alle Aufbereitungen von reinen Pixeldaten und die von diesen oder von den Pixeldaten abgeleiteten Daten verstanden werden, insbesondere sogenannte vektororientierte Bilddaten.

Wenn beispielsweise Bestandsdaten des Patienten herangezogen werden, dann können diese in der Form einer Diagnose oder Indikation oder von früher durch das System selbst oder auch durch eine andere bildgebende Einrichtung aufgenommenen Aufnahmen der Netzhaut des Patienten vorliegen. Diese sollen dann in dem System mit einer aktuellen Aufnahme abgeglichen werden können. Bestandsdaten können auch Daten einer früher an dem Patienten vorgenommenen Behandlung oder Daten zu einem Behandlungsstatus sein. Grundsätzlich können Bestandsdaten alle bereits vor Behandlungsbeginn oder vor Beginn der Erstellung des Planungsdatensatzes schriftlich oder in elektronisch, insbesondere digital, gespeicherter Form verfügbaren Daten des Patienten sein. Diese können dem System über elektronische Schnittstellen übermittelt oder manuell eingegeben werden. Die Bestandsdaten können beispielsweise in einem umfassenderen System in elektronischer Form vorliegen, von dem das erfindungsgemäße System zur Erstellung von Planungsdatensätzen ein Untersystem oder Teilsystem darstellt.

Ein Vorteil einer Kamera, die aktuelle Aufnahmen der Netzhaut erfasst, ergibt sich, wenn mit dem System zur Erstellung von Planungsdaten auch eine Behandlungsvorrichtung kombiniert ist, die einen Behandlungslaser aufweist, der relativ zu der Kamera justiert oder mit dieser abgeglichen ist, um unter Anwendung der Planungsdaten die Behandlung durchzuführen. Die Strukturierung der Planungsdaten wird weiter unten im Detail erläutert.

Weiter ist eine Bildverarbeitungseinrichtung vorgesehen, die aus den aktuell erfassten Bilddaten der Kamera strukturierte Bilddaten der Netzhaut erzeugt und diese für die Planung bereitstellt. Unter strukturierten Bilddaten sind beispielsweise Daten zu verstehen, die bestimmte in den Aufnahmen durch die Bildverarbeitung erkannte Objekte oder auch Eigenschaften der Aufnahme beziehungsweise der Netzhaut oder Eigenschaften von Aufnahmebereichen oder Teilen der Netzhaut enthalten oder darstellen. Beispielsweise können die strukturierten Bilddaten Helligkeits-oder Farbverteilungen, Intensitätsverteilungen auf einem Infrarotbild, die Position, Form und/oder Verläufe von Objekten oder Objektgrenzen beschreiben. Die Repräsentation solcher Strukturen in Form von Daten kann vielfältig sein und beispielsweise reine Bilddaten, aber auch vektorielle Darstellungen, Vektoren, Matrizen oder andere Arten von abstrahierten Repräsentationen enthalten. Beispielsweise sollen die strukturierten Daten in übersichtlicher Form, beispielsweise auch vektororientiert oder mit Metadaten, die sich auf geometrische Formen beziehen, die Form, Lage und gegebenenfalls weitere Parameter von Blutgefäßen, Nervenknoten und allgemein von Auffälligkeiten auf der Netzhaut beschreiben.

Die Lage von solchen Objekten, die auf der Netzhaut aktuell erkannt werden, kann und soll gegebenenfalls bei der Erstellung eines Behandlungsplans und der Erzeugung entsprechender Planungsdaten berücksichtigt werden.

Inwieweit solche aktuell erfassten strukturierten Bilddaten berücksichtigt werden, kann unter anderem von den Bestandsdaten des Patienten abhängen. Unter den Bestandsdaten werden dabei, wie oben ausgeführt, solche Daten verstanden, die sich auf den Patienten beziehen und bereits vorliegen und verfügbar sind, wenn das System auf den Patienten angewendet wird.

Es kann bei dem System vorgesehen sein, dass bestimmte Elemente des Systems lokal an dem Ort der Behandlung des Patienten vorhanden sind, während andere Elemente an einem anderen Ort liegen oder schwierig lokalisierbar sind. Beispielsweise kann die Bildverarbeitungseinrichtung oder auch die Verarbeitungseinrichtung für die Erzeugung von Planungsdaten oder Teile dieser Einrichtung, wie beispielsweise Speichereinrichtungen, an einem anderen Ort lokalisiert sein als der Behandlungslaser. Datenverarbeitungseinrichtungen können beispielsweise in größeren Computern oder Datenverarbeitungsanlagen liegen als am Ort der Laserbehandlung verfügbar sind. Wenn die Verarbeitungseinrichtungen Zugriff auf viele gespeicherte Daten brauchen, können sowohl die Verarbeitungseinrichtungen als auch Speichereinrichtungen auf mehrere Computer verteilt oder in einer größeren, zentralen Computeranlage verortet sein. Beispielsweise kann es sinnvoll sein, Behandlungsdaten vieler Patienten zentral oder in einem verteilten Rechnersystem in Form einer Datenbank zu speichern, um bei der Interpretation von individuellen Daten eines einzelnen Patienten oder auch für das Trainieren einer lernenden Einrichtung des Systems möglichst viele Daten verfügbar zu haben.

Einzelne Teile oder Module des Systems, wie beispielsweise die Bestandsdatenerfassungseinrichtung, eine Messdatenerfassungseinrichtung oder eine Änderungseinrichtung zur manuellen Korrektur von Planungsdaten oder auch eine Einrichtung zur Eingabe von Daten, die den Behandlungserfolg beschreiben, können beispielsweise auch als Applikation auf einem mobilen Endgerät installiert sein. Die Datenformate der Übermittlung von Eingangsdaten an das mobile Gerät und der Ausgabe von dem mobilen Gerät an einen stationären Teil des Systems, der auch die Kamera und/oder die Verarbeitungseinrichtung enthält, erfolgt dann über Schnittstellen, bei denen sowohl die Datenformate als auch die Datenübermittlungswege, wie zum Beispiel hardware- Anschlüsse, Datenbus oder Funkschnittstelle, eindeutig definiert sind, um ein integriertes System zu erhalten.

Eine vorteilhafte Ausgestaltung eines solchen Systems kann vorsehen, dass die Bestandsdatenerfassungseinrichtung dazu eingerichtet ist, Diagnosedaten und/oder Indikationsdaten und/oder Daten einer durchgeführten Vorbehandlung und/oder vorbekannte physiologische Daten des Patienten und/oder bereits vorhandene, außerhalb des Systems gespeicherte Aufnahmen der Netzhaut des Patienten zu erfassen und bereitzustellen.

Solche Bestandsdaten können beispielsweise in der Form einer elektronischen Datei vorliegen, manuell eingebbar oder einlesbar sein, beispielsweise eine Diagnose in Text- oder Bildform oder eine Indikation, die bereits Informationen zu einer empfohlenen Behandlung gibt. Solche Informationen können bereits Daten enthalten, die bestimmte Orte oder Bereiche der Netzhaut differenzieren und ortsabhängig vom jeweiligen Ort auf der Netzhaut sind, wie beispielsweise die genaue Verortung von zu behandelnden Stellen oder Bereichen. Solche Informationen können aber auch ortsunabhängig sein und den Zustand des Patienten allgemein, seiner Augen oder andere Eigenschaften, beispielsweise physiologische Daten des Patienten beschreiben. Solche Daten können in der Form von älteren Bildern der Netzhaut unterschiedlicher Art, beispielsweise Farbbildern, Schwarzweißbildern, Infrarotbildern, Fluoreszenzbildern oder Tomografiebildern, vorliegen, oder in Altersangaben, Hautfarbe oder Geschlecht des Patienten, einer Identität des Patienten oder Informationen über Vorbehandlungen bestehen, die mit oder ohne Einbindung des erfindungsgemäßen Systems stattgefunden haben können.

Weiter kann ein solches System in einer Ausführungsform derart ausgestaltet sein, dass eine Messdatenerfassungseinrichtung vorgesehen ist, die eine oder mehrere Sensoreinrichtungen zur Erfassung und Bereitstellung von physiologischen Daten des Patienten, insbesondere Daten, die vom jeweiligen Ort auf der Netzhaut abhängen, und/oder Daten, die nicht von einem Ort auf der Netzhaut abhängen, aufweist, und dass die Verarbeitungseinrichtung dazu eingerichtet ist, bei der Zuordnung von Planungsdaten die erfassten physiologischen Daten des Patienten zu berücksichtigen.

Solche Messdatenerfassungsvorrichtungen können grundsätzlich alle Arten von Vorrichtungen sein, die die aktuelle Erfassung von zusätzlichen Informationen über den Patienten und seine physiologischen Daten ermöglichen. Dazu gehören Sensoreinrichtungen wie Kameras, die andere Eigenschaften aufnehmen als die eingangs genannte Kamera, also beispielsweise andere bildgebende Verfahren oder auch ähnliche oder dasselbe bildgebende Verfahren wie die eingangs für die Aufnahme der Netzhaut genannte Kamera anwenden. Mit solchen Kameras erfasste Aufnahmen der Netzhaut stellen dann Daten bereit, die vom jeweiligen Ort der Netzhaut abhängig sind. Beispielsweise kann mit einer Sensoreinrichtung die Temperatur oder Temperaturverteilung auf der Netzhaut gemessen werden. Mittels Aufnahmen der Netzhaut kann beispielsweise unter Verwendung von Algorithmen auch eine Erkrankung der Netzhaut erkannt werden, die für die Erstellung von Planungsdaten berücksichtigt werden kann. Es kann auch eine Vorrichtung zur Erstellung einer OCT-Aufnahme oder eine Vorrichtung zur Reflektometrie vorgesehen sein. Mit einer Kamera, insbesondere auch mit derselben Kamera, die dazu eingerichtet ist, laufend Aufnahmen der Netzhaut zu erfassen, können auch Reaktionen der Netzhaut auf Titrationsschüsse, das heißt versuchsweise Anwendungen des Behandlungslasers, erfasst werden, um die Laserparameter aufgrund dieser Reaktionen planen und steuern zu können oder zumindest diese Reaktionen bei der Erzeugung von Planungsdaten berücksichtigen zu können. Es sind zudem auch Sensoreinrichtungen denkbar, die andere physiologische Eigenschaften des Patienten messen, wie beispielsweise die Hautfarbe und/oder - helligkeit, Hautdicke, Haarfarbe, Farbe und/oder Farbverteilung der Iris, Blutdruck, verschiedene Blutwerte oder Ähnliches. Auch diese Sensoreinrichtungen können beispielsweise eine Kamera oder optische Sensoren umfassen.

Eine besondere Implementierung des Systems kann beispielsweise auch vorsehen, dass die oben genannten Messdatenerfassungseinrichtungen auch nach einer ersten Erzeugung von Planungsdaten für eine Laserbehandlung und gegebenenfalls auch nach einer manuellen Korrektur von Planungsdaten noch während der eigentlichen Laserbehandlung weiter zur Erfassung von Daten betrieben werden. Beispielsweise kann die Temperatur der Netzhaut oder Temperaturverteilung auf der Netzhaut durch eine solche Messeinrichtung auch während der Behandlung oder in Behandlungspausen laufend weiter erfasst werden. Die so gewonnenen Messdaten können eine Änderung der Planungsdaten erfordern oder vorteilhaft machen. Daher kann es vorgesehen sein, dass aufgrund der nach der ersten Erstellung eines Planungsdatensatzes ermittelten Messdaten durch das System eine weitere, korrigierte Planungsdatei erzeugt und der weiteren Behandlung zugrunde gelegt wird.

Das System kann auch beispielsweise weitergebildet sein durch ein Anzeigemodul oder eine Anzeigeeinrichtung, das/die die grafische Darstellung eines Planungsdatensatzes innerhalb einer durch die Kamera aktuell erfassten Aufnahme der Netzhaut bewirkt. Auf diese Weise können die Planungsdaten gemeinsam mit einer grafischen Darstellung einer Aufnahme der Netzhaut visualisiert werden, so dass beispielsweise unmittelbar die Berücksichtigung der strukturierten Bilddaten beurteilt werden kann, indem sichtbar wird, ob zum Beispiel bestimmte sensible Bereiche der Netzhaut, wie Blutgefäße oder Nervenknoten, bei der Behandlung ausgenommen sind oder ob bestimmte, bereits im aktuellen Bild sichtbare und als behandlungsbedürftig eingestufte Bereiche im Behandlungsplan angemessen berücksichtigt sind.

Zudem kann, wenn eine Behandlungseinrichtung in das System integriert ist und wenn das aktuelle Kamerabild mit der Lenkungseinrichtung für einen Behandlungslaser abgeglichen wird, damit auch der Planungsdatensatz direkt mit der Lenkungseinrichtung abgeglichen werden.

Das System kann auch durch eine Trackingeinrichtung weitergebildet sein, die bei einer Bewegung, insbesondere einer Verschiebung oder Rotation der aktuell durch die Kamera erfassten Aufnahme eine Nachführung der Darstellung des Planungsdatensatzes bewirkt. Eine solche Verschiebung der Aufnahme kann beispielsweise bei einer Bewegung des Patienten gegenüber der Kamera oder bei einer Augenbewegung auftreten, und die Trackingeinrichtung bewirkt in einem solchen Fall, dass die Darstellung der Planungsdaten weiterhin zum angezeigten aktuellen Kamerabild passt. Wenn eine Behandlungseinrichtung in das System integriert ist, kann durch eine solche Nachführung auch sichergestellt sein, dass die Lenkungseinrichtung für einen Behandlungslaser planungsgemäß ausgerichtet bleibt.

Zudem kann bei einem System der genannten Art vorgesehen sein, dass die Verarbeitungseinrichtung eine Speichereinrichtung aufweist, die zur Speicherung von Bestandsdaten von Patienten und von diesen zugeordneten Planungsdatensätzen eingerichtet ist, sowie insbesondere zusätzlich zur Speicherung von jeweils zu den vorgenannten Daten zugehörigen, durch eine Messdatenerfassungseinrichtung erfassten physiologischen Daten und/oder zur Speicherung von jeweils vorgenommenen manuellen Änderungen der Planungsdatensätze.

Somit wird dem System ermöglicht, die Zuordnung eines aktuellen Planungsdatensatzes zu den Bestandsdaten eines Patienten mit früheren Zuordnungen bei ähnlichen oder sich unterscheidenden Bestandsdaten zu vergleichen. Damit kann beispielsweise eine Plausibilitätsanalyse durchgeführt werden. Es können, wenn in früheren Fällen später manuell Planungsänderungen vorgenommen worden sind, auch diese berücksichtigt und beispielsweise bei der Planung vorweggenommen werden. Zudem können, falls auch erfasste physiologische Daten gespeichert sind, auch diese beim Vergleich und bei einem etwaigen Abgleich der zu erstellenden Planungsdatei mit früheren Planungen berücksichtigt werden. Demgemäß kann das erfindungsgemäße System als Expertensystem oder auch als lernendes System ausgestaltet sein.

Weiter kann bei einem solchen System vorgesehen sein, dass die Verarbeitungseinrichtung eine Speichereinrichtung aufweist, die zur Speicherung von Bestandsdaten von Patienten sowie von mit dem System erzeugten strukturierten Bilddaten und/oder Aufnahmen der Netzhaut des Patienten und von diesen zugeordneten Planungsdatensätzen insbesondere unter anderem Zielwerte zu Messdaten, eingerichtet ist, sowie insbesondere zusätzlich zur Speicherung von jeweils zu den vorgenannten Daten zugehörigen, durch eine Messdatenerfassungseinrichtung erfassten physiologischen Daten und/oder zur Speicherung von jeweils vorgenommenen manuellen Änderungen der Planungsdatensätze.

Diese Ausführungsform des Systems ermöglicht es, die Zuordnung eines aktuellen Planungsdatensatzes zu den Bestandsdaten eines Patienten und den strukturierten Bilddaten mit früheren Zuordnungen bei ähnlichen oder sich unterscheidenden Bestandsdaten sowie ebenfalls gespeicherten strukturierten Bilddaten zu vergleichen. Auch mit diesen Daten kann eine Plausibilitätsanalyse durchgeführt werden. Es können, wenn in früheren Fällen später manuell Planungsänderungen vorgenommen worden sind, auch diese berücksichtigt und beispielsweise bei der Planung vorweggenommen werden. Zudem können in dem so gebildeten Expertensystem, falls auch erfasste physiologische Daten gespeichert sind, auch diese beim Vergleich und bei einem etwaigen Abgleich der zu erstellenden Planungsdatei mit früheren Planungen berücksichtigt werden.

Eine weitere Weiterbildung des Systems kann beispielsweise durch eine Überprüfungseinrichtung, auch Monitoringeinrichtung genannt, gegeben sein, die nach Durchführung einer Behandlung der Netzhaut Daten zu den Auswirkungen der Behandlung erfasst und bereitstellt.

Dabei kann die Behandlung der Netzhaut eine Laserbehandlung umfassen, jedoch auch zusätzlich noch weitere Behandlungsschritte mit oder ohne Verwendung eines Lasers. Die Überprüfung kann unmittelbar während der Behandlung oder nach Abschluss der Behandlung, insbesondere auch eine bestimmte Mindestzeit nach der Behandlung, durchgeführt werden. Die Überprüfungseinrichtung kann eine Einrichtung zur Durchführung eines bildgebenden Verfahrens umfassen, beispielsweise auch unter Verwendung der eingangs genannten Kamera zur Erfassung aktueller Aufnahmen der Netzhaut. Die Überprüfungseinrichtung kann auch eine Eingabeeinrichtung zur manuellen Eingabe von Daten umfassen. Dabei kann vorgesehen sein, dass nur eine binäre Information wie "Behandlung erfolgreich" oder "Behandlung nicht erfolgreich" erfasst wird oder eine Bewertung durch den Patienten, wie zum Beispiel "habe Schmerzen, ja/nein" oder eine weitergehende Beurteilung, zum Beispiel, welche Parameter der Behandlung, wie z. B. die Laserparameter, ausgewählte geometrische Behandlungsmuster (patterns), zur Behandlung vorgesehene oder von der Behandlung ausgenommene Bereiche, nach Meinung einer beurteilenden Person suboptimal waren. Dazu können bestimmte Kategorien der Beurteilung vorgegeben sein, wie beispielsweise ein Bewertungsschema. Auch eine solche Bewertung kann in die oben beschriebenen Expertensysteme aufgenommen, gemeinsam mit den gespeicherten Planungsdatensätzen abgespeichert und beim Abgleich aktueller Planungsdaten mit gespeicherten Planungsdaten berücksichtigt werden.

Die durch das System erzeugten Planungsdatensätze können in einer Implementierung des Systems Planungsdaten von einer, zwei, drei, vier oder fünf der folgenden Kategorien enthalten: Laserparameter, Ortsdaten für die Laserbehandlung, Behandlungsmuster, Ortsdaten für Bereiche, die von der Laserbehandlung ausgeschlossen sind, sowie Zielwerte für Messwerte, die während einer nachfolgenden Behandlung durch die Messwerterfassungseinrichtung oder anderweitig erfasst werden. Derartige Zielwerte können beispielsweise eine mittlere oder auch vom Ort auf der Netzhaut abhängige Zieltemperatur sein, die während der Laserbehandlung laufend erfasst wird und die beispielswiese erreicht, eingehalten oder nicht überschritten werden soll. Die übrigen Behandlungsparameter können dann während der Behandlung dynamisch so angepasst werden, dass die vorgegebenen Zielwerte erreicht oder nicht überschritten werden. Es werden somit in den Planungsdatensätzen beispielsweise Parameter für eine Regelung vorgegeben. Zudem können als eine weitere Kategorie von Planungsdaten auch Berichtsvorlagen für die Behandlung mit erzeugt werden, das heißt beispielsweise Formulare sowie die Auswahl und Darstellung der zu berichteten Daten und Parameter.

Die Erfindung kann weiter vorteilhaft dadurch realisiert sein, dass die Verarbeitungseinrichtung als selbst lernende Einrichtung ausgebildet ist, in der die Zuordnungsfunktion, die den Bestandsdaten und insbesondere den strukturierten Bilddaten sowie insbesondere gemessenen physiologischen Daten eines Patienten Planungsdaten zuordnet, dadurch trainiert wird, dass ihr jeweils zu einem Patienten einerseits zumindest Daten von wenigstens einer der folgenden Kategorien: Bestandsdaten, strukturierte Bilddaten, gemessene physiologische Daten, und andererseits zugeordnete Planungsdaten, insbesondere unter anderem Zielwerte zu Messdaten, sowie manuelle Planungsdatenänderungen und insbesondere Daten zu einer nach Durchführung der geplanten Behandlung erfassten Auswirkung der Behandlung als Trainingsdaten vorgegeben werden.

Die Planungsdatensätze, die das System erzeugt, können dabei Planungsdaten von einer, zwei, drei, vier oder fünf der folgenden Kategorien enthalten: Laserparameter, Ortsdaten für die Laserbehandlung, Behandlungsmuster, Ortsdaten für Bereiche, die von der Laserbehandlung ausgeschlossen sind, Zielwerte für Messdaten. Dabei können die Laserparameter beispielsweise die Laserleistung, die Wellenlänge, Pulsdauer, Wiederholungsrate der Pulse und die Gesamtzahl der Laserpulse sowie die Größe des Lichtflecks des Lasers auf der Netzhaut sowie jede einzelne dieser Größen oder alle denkbaren Unterkombinationen umfassen. Diese Laserparameter können jeweils vom Ort oder Bereich auf der Netzhaut abhängig sein. Die Ortsdaten der Laserbehandlung können einen oder mehrere geometrische Bereiche auf der Netzhaut umfassen. Auch die von der Behandlung ausgeschlossenen Bereiche der Netzhaut können einen oder mehrere geometrische Bereiche der Netzhaut umfassen. Die Behandlungsmuster können geometrische Muster, wie beispielsweise Kreise, Ellipsen, Rechtecke, Dreiecke oder beliebige Freiformen umfassen, in denen die Laserbehandlung beispielsweise mit gleichbleibenden Laserparametern stattfinden soll oder in denen einer der Laserparameter gleichbleibt. Diese Behandlungsmuster können auch Flächen sein, die nach einem Startimpuls durch den Operateur durch den Behandlungslaser selbständig abgearbeitet, das heißt bestrahlt werden. Die Behandlungsmuster können auch einfach geometrische Bereiche umfassen, die der Laser in einem Zug, beispielsweise Punkt für Punkt nach einem Punktemuster, abarbeitet, ohne dass der Operateur jeden einzelnen Punkt ansteuern muss. Es kann lediglich ein Startpunkt manuell oder automatisch angesteuert werden, und der Rest eines Behandlungsmusters kann dann automatisch gesteuert von der Lasersteuerung abgefahren werden. Die Dichte der anzufahrenden Punkte kann ebenfalls einer der Parameter der Kategorie "Behandlungsmuster" (patterns) sein.Planungsdaten können auch Zielwerte für Messdaten sein, die während der Behandlung erfasst werden, wie beispielsweise eine auf der Netzhaut während der Behandlung zu erreichende oder nicht zu überschreitende Temperatur, die insgesamt als Durchschnittswert oder ortsabhängig vorgegeben werden kann und die während der Behandlung laufend erfasst/gemessen wird. Das Behandlungssystem wird dann so geregelt, dass die Zielwerte eingehalten werden. Als Zielwerte können auch laufend erfassbare optische Größen, wie eine mittels einer Kamera oder eines Hyperspektralsensors erfasste Reflektionsänderung Verfärbung/spektrale Veränderung oder Helligkeitsänderung der jeweils behandelten Bereiche vorgegeben werden.

Jede dieser genannten Kategorien von Planungsdaten, oder genauer gesagt die Daten jeder einzelnen der genannten Kategorien, kann/können, gegebenenfalls mit Einschränkungen wie oben ausgeführt, manuell von einer Person, insbesondere von dem jeweiligen Operateur, geändert werden. Dieser kann die im ersten Versuch von dem System erzeugten Planungsdaten mit den von der Kamera aktuell erfassten Bilddaten oder den strukturierten Daten sowie mit seinen Erfahrungswerten abgleichen und gemäß seiner Einschätzung abändern.

Die von der Behandlung auszunehmenden Bereiche können dabei eine besondere Rolle spielen. Sie können sich gedanklich zwar in vielen Fällen als Komplementärmenge der Ortsdaten für die Laserbehandlung ergeben, jedoch ist es in vielen Fällen weniger fehleranfällig, die von der Behandlung auszunehmenden Bereiche der Netzhaut anzugeben als die Ortsdaten für die Behandlung, insbesondere beispielsweise dann, wenn die Netzhaut gleichförmig bis auf wenige auszunehmende Bereiche zu bearbeiten ist. Zudem können die auszunehmenden Bereiche sehr sicherheitsrelevant sein, da darunter auch Bereiche fallen, in denen durch eine Laserbehandlung gesundheitlicher Schaden entstehen kann. Es kann deshalb beispielsweise auch vorgesehen sein, die gemäß der von dem System erzeugten Planungsdaten von der Behandlung auszunehmenden Bereiche derart festzuschreiben, dass diese manuell nicht geändert werden können oder dass für ihre Änderung bestimmte Hindernisse zu überwinden oder Berechtigungen nachzuweisen sind.

Das System kann als selbstlernendes System ausgebildet sein, das anhand der oben genannten Daten, die ihm zur Verfügung gestellt werden, den Zuordnungsalgorithmus, der die Erzeugung von Planungsdaten bewirkt, laufend verbessert. Dabei kann das System ein neuronales Netz aufweisen, das trainiert wird, oder einen analytischen, statistischen oder parametrischen Lernalgorithmus aufweisen, statisch, geführt lernend oder selbstlernend sein. Der Zuordnungsalgorithmus kann zusätzlich auch analytische Elemente aufweisen, die durch den Lernvorgang nicht veränderbar sind und mit Expertenwissen vorgegeben werden, wie zum Beispiel bestimmte Bedingungen, die die Laserparameter erfüllen müssen, beispielsweise Leistungsbegrenzungen oder Vorschriften, denen die Ausschlüsse von Behandlungsorten folgen müssen.

Für den Lernvorgang aufgrund der zur Verfügung stehenden Behandlungsdaten, der regelmäßig bei einer Erweiterung und Vermehrung der zur Verfügung stehenden Behandlungsdaten durchgeführt werden kann, können die Elemente des Lernvorganges beispielsweise geordnet und gewichtet werden. Beispielsweise können die Daten, die auf den nach der Laserbehandlung mit der Monitoringeinrichtung ermittelten Behandlungserfolgen basieren, eine höhere Gewichtung erhalten als die manuellen Korrekturen von Planungsdaten, die von behandelnden Personen vorgenommen werden. Es können auch beispielsweise Bedingungen eingeführt werden, die erfüllt sein müssen, damit Behandlungsdaten dem lernenden System beziehungsweise dem Lernalgorithmus zur Verfügung gestellt werden. Beispielsweise kann eine solche Bedingung darin bestehen, dass bestimmte manuelle Parameteränderungen, beispielsweise der Laserparameter, innerhalb einer festgelegten Bandbreite in einer bestimmten Mindestanzahl von Fällen oder durch eine Mindestanzahl von behandelnden Personen oder an einer Mindestanzahl von Behandlungsorten vorgenommen worden sein müssen, damit diese Änderungen dem lernenden System als Trainingsdaten vorgegeben werden.

Die Erfindung bezieht sich außer auf ein System der oben beschriebenen Art auch auf eine Vorrichtung zur Erstellung von Planungsdatensätzen für die Gewebebehandlung der Netzhaut eines Auges mittels eines Laserstrahls,
- mit einer Kamera, die dazu eingerichtet ist, laufend Aufnahmen der Netzhaut zu erfassen,
- mit einer Bildverarbeitungseinrichtung, die aus durch die Kamera erfassten Aufnahmen aktuelle strukturierte Bilddaten der Netzhaut erzeugt und bereitstellt,
- mit einer Bestandsdatenerfassungseinrichtung, die dazu eingerichtet ist, Bestandsdaten eines Patienten zu erfassen und/oder bereitzustellen,
und mit einer Verarbeitungseinrichtung, die dazu eingerichtet ist, den aktuellen strukturierten Bilddaten der Netzhaut und den Bestandsdaten des Patienten Planungsdatensätze zuzuordnen, wobei in der Verarbeitungseinrichtung Vorgaben, insbesondere Muster und Regeln, für die Zuordnung von Planungsdaten zu strukturierten Bilddaten und Bestandsdaten von Patienten gespeichert sind.Die Vorrichtung kann vorteilhaft zusätzlich eine Änderungseinrichtung aufweisen, die die manuelle Änderung eines Planungsdatensatzes ermöglicht.

Außer auf ein System und eine Vorrichtung bezieht sich die Erfindung auch auf ein Verfahren zur Erstellung von Planungsdatensätzen für die Gewebebehandlung der Netzhaut eines Auges eines Patienten mittels eines Laserstrahls,
- bei dem mit einer Kamera wenigstens eine Aufnahme der Netzhaut erfasst wird,
- bei dem mit einer Bildverarbeitungseinrichtung aus durch die Kamera erfassten Aufnahmen aktuelle strukturierte Bilddaten der Netzhaut erzeugt und bereitgestellt werden,
- bei dem im Rahmen einer Bestandsdatenerfassungseinrichtung Bestandsdaten eines Patienten erfasst und/oder bereitgestellt werden,
- und bei dem durch eine Verarbeitungseinrichtung den aktuellen strukturierten Bilddaten der Netzhaut und den vorbekannten Bestandsdaten des Patienten aufgrund von Vorgaben, insbesondere Mustern und Regeln, die in der Verarbeitungseinrichtung gespeichert sind, Planungsdatensätze zugeordnet werden.

Das Verfahren kann beispielsweise dadurch vorteilhaft ausgestaltet sein, dass zugeordnete Planungsdatensätze nach der Zuordnung manuell geändert werden.

Weiter kann das Verfahren dadurch vorteilhaft verwirklicht werden, dass physiologische Daten des Patienten insbesondere mit einer Messdatenerfassungseinrichtung erfasst und bereitgestellt sowie bei der Zuordnung von Planungsdatensätzen durch die Verarbeitungseinrichtung berücksichtigt werden. Die physiologischen Daten können, insbesondere wenn es sich um leicht erkennbare Eigenschaften des Patienten, wie die Haar- oder Irisfarbe, handelt, auch durch eine Person erfasst und eingegeben werden.

Das Verfahren kann außerdem dadurch vorteilhaft verwirklicht werden, dass die Verarbeitungseinrichtung als selbstlernende Einrichtung ausgebildet ist und die Zuordnungsfunktion, die den Bestandsdaten und den strukturierten Bilddaten sowie insbesondere durch eine Messdatenerfassungseinrichtung gemessenen physiologischen Daten eines Patienten Planungsdaten zuordnet, dadurch trainiert wird, dass ihr zu einer Mehrzahl von Patienten jeweils die Bestandsdaten, strukturierte Bilddaten, zugeordnete Planungsdaten, Daten der manuellen Planungsdatenänderungen sowie insbesondere gemessene oder erfasste physiologische Daten und/oder Daten zu der nach Durchführung der jeweils geplanten Behandlung erfassten Auswirkung der Behandlung vorgegeben werden.

Das Verfahren kann dabei, ebenso wie das oben beschriebene System, so gestaltet werden, dass als Planungsdatensätze Planungsdaten von einer, zwei, drei, vier oder fünf der folgenden Kategorien erzeugt werden: Laserparameter, Ortsdaten für die Laserbehandlung, Behandlungsmuster, Ortsdaten für Bereiche, die von der Laserbehandlung ausgeschlossen sind, Zielwerte für Messdaten.

Die verschiedenen Arten, Erscheinungs- und Darstellungsformen der einzelnen Arten von Planungsdaten sind weiter oben bereits ausführlich beschrieben worden.

Die Erfindung kann sich zudem auf ein Verfahren zum Betrieb eines Systems und/oder einer Vorrichtung der oben beschriebenen Art beziehen, bei dem ein Planungsdatensatz erstellt und dann in einer separaten oder einer in das System oder die Vorrichtung integrierten Behandlungseinrichtung mittels eines Behandlungslasers eine Behandlung gemäß dem Planungsdatensatz durchgeführt wird.

Danach kann beispielsweise noch eine Beurteilung des Erfolges der Behandlung mittels einer Monitoringeinrichtung erfolgen und Daten, die den Erfolg repräsentieren, können dem System in digitaler Form zugeführt werden, um ein Lernen des Systems zu ermöglichen.

Die Erfindung wird anhand von Ausführungsbeispielen in Figuren gezeigt und nachfolgend erläutert. Dabei zeigt
- Fig. 1: wesentliche Teile der erfindungsgemäßen Vorrichtung und des Systems mit einer in ein Ophthalmoskop integrierten und mit einem Behandlungslaser kombinierten Kamera, einer Bildverarbeitungseinrichtung, einer Verarbeitungseinrichtung zur Erzeugung von Planungsdaten sowie einigen weiteren Datenerfassungs-und Eingabeeinrichtungen,
- Fig. 2: schematisch ein System zur Erstellung von Planungsdatensätzen,
- Fig. 3: schematisch ein System zur Erstellung von Planungsdatensätzen mit einem Lernalgorithmus, sowie
- Fig. 4: ein beispielhaftes Ablaufdiagramm für das erfindungsgemäße Verfahren.

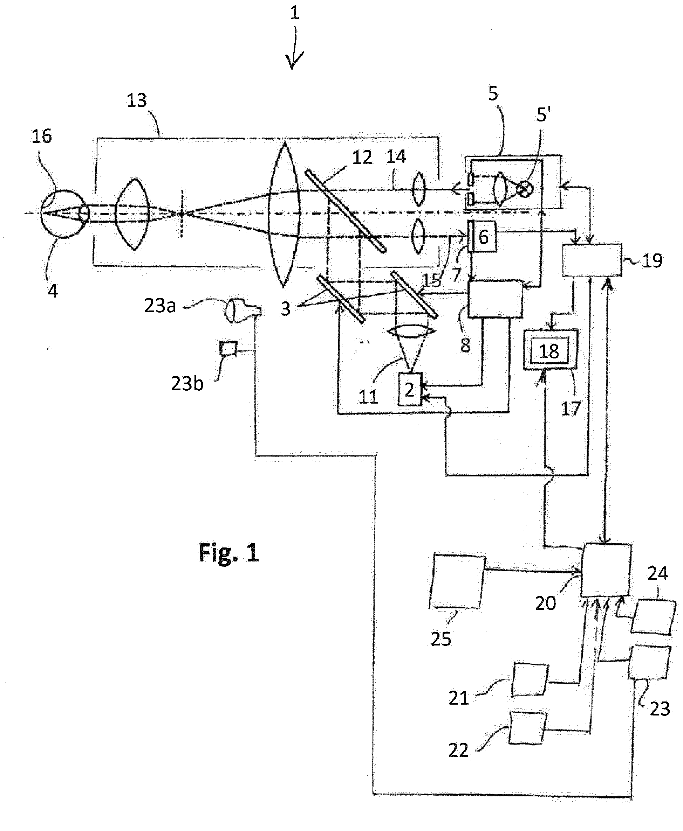

Figur 1 zeigt zumindest teilweise eine Vorrichtung und ein System gemäß der Erfindung mit einem schematisierten Ophthalmoskop 1, das in dem System zur Durchführung des erfindungsgemäßen Verfahrens verwendet werden kann und die Funktionen eines Ophthalmoskops mit einer Kamera mit Funktionen der Laserbehandlung der Netzhaut verbindet.

Die Vorrichtung weist eine Beleuchtungseinrichtung 5 mit einer Strahlungsquelle 5' auf, die dazu eingerichtet ist, einen Beleuchtungsstrahl 14 auf das Auge 4 und die Netzhaut 16 eines Patienten zu richten. Damit kann die Netzhaut 16 zur Erfassung einer Aufnahme beziehungsweise eines Kamerabilds geeignet beleuchtet werden. Die Beleuchtung kann beispielsweise mit einer Leuchtdiode oder einer Infrarotdiode als Lichtquelle ausgestattet sein, oder mit einer Lichtquelle anderer Art, die ein gewünschtes Wellenlängenspektrum liefert. Die Lichtquelle kann beispielsweise auch eine UV-Lichtquelle sein.

Das Ophthalmoskop verfügt zudem über eine Kamera 6, bei der ein Bildsensor mit 7 bezeichnet ist. Der Sensor kann beispielsweise ein CCD-Sensor sein. Anstelle der Kamera 6 kann auch jede andere Art von Einrichtung vorgesehen sein, die beispielsweise als Scanvorrichtung dazu geeignet ist, Strahlung, die von der Netzhaut auf einen Sensor gelangt, zu detektieren und eine Aufnahme der Netzhaut zu erzeugen. Das Ziel beim Betrieb der Beleuchtungseinrichtung 5 und der Kamera 6 oder einer gleichwertigen Einrichtung besteht darin, durch Erfassung einer Aufnahme von rückgestrahlter Strahlung möglichst genaue, ortsaufgelöste Messdaten von der Netzhaut 16 zu erhalten und damit die Eigenschaften der gesamten Netzhaut oder von zu behandelnden Zielbereichen zu erfassen beziehungsweise aus erfassten Daten ermitteln zu können.

Der Beleuchtungsstrahl 14 und die zurückgeworfene Strahlung 15 werden durch ein geeignetes optisches System 13 mit Spiegeln und Linsen in an sich bekannter Weise geeignet kollimiert beziehungsweise fokussiert. Das optische System 13 weist zudem einen Strahlteiler 12 auf, der es ermöglicht, einen Laserstrahl 11 von dem Behandlungslaser 2 auf die Netzhaut 16 zu richten. Anstelle eines Strahlteilers können auch verschiedene Strahlen des Behandlungslasers und der Beleuchtung und/oder Bilderfassung in einem gewissen Abstand parallel zueinander geführt werden.

Zur Steuerung des Lasers 2 kann eine Steuereinheit 8 vorgesehen sein, die einerseits die Beleuchtungseinrichtung 5 steuert, beispielsweise triggert, andererseits auf der Basis eines Kamerabildes der Kamera 6 unmittelbar den Laser 2 steuert. Die Steuereinheit 8 kann dabei auch Umlenkspiegel 3 steuern, die den Strahlengang des Behandlungsstrahls 11 lenken und damit gezielt die Behandlung einzelner Zielbereiche auf der Netzhaut 16 ermöglichen. In dem erfindungsgemäßen System ist zudem eine Bildverarbeitungseinrichtung 19 vorgesehen, die eine digitale Verarbeitung von Aufnahmen/Kamerabildern der Kamera 6 erlaubt und aus den Bilddaten, die beispielsweise in Form von Pixeldaten vorliegen, strukturierte Daten ermittelt, die zum Beispiel in Form einer Vektorgrafik oder in einer anderen, strukturierten Form bereitgestellt werden können. Die Bilddaten und/oder strukturierte Bilddaten können auch der Steuereinheit 8 zur Verfügung gestellt werden. Die Bildverarbeitungseinrichtung 19 stellt die Daten auch für die Anzeigeeinrichtung 17 bereit, die einen Bildschirm 18 aufweist, auf dem beispielsweise ein Abbild der Netzhaut dargestellt wird. In der Anzeigeeinrichtung 17 können zusätzlich zu den Bilddaten der Kamera 6 und/oder strukturierten Bilddaten der Bildverarbeitungseinrichtung 19 auch Planungsdaten verarbeitet und gemeinsam mit den Bilddaten in numerischer oder grafischer Darstellung angezeigt werden. Somit können beispielsweise in den aktuellen Bilddaten, das heißt in einer Live-Ansicht der Netzhaut, Behandlungsbereiche markiert und/oder Behandlungsmuster oder auch Laserparameter angezeigt werden. In der Anzeigeeinrichtung oder verbunden mit dieser ist auch eine Tracking-Einrichtung vorgesehen, die im Falle einer Bewegung des Kamerabildes, das heißt einer Bewegung von in dem Kamerabild dargestellten Strukturen, die Elemente der Planungsdaten, die den Bilddaten zugeordnet sind, mit verschiebt.

In Figur 1 ist als Teil der Vorrichtung und des Systems auch die Verarbeitungseinrichtung 20 gezeigt, die Planungsdateien beziehungsweise Planungsdaten der Laserbehandlung erzeugt und dazu zumindest Bestandsdaten aus einem Bestandsdatenspeicher 21 oder mittels einer ersten Eingabeeinrichtung 22 eingegebene Bestandsdaten des zu behandelnden Patienten sowie strukturierte Kamerabilder nutzt. Zusätzlich kann die Verarbeitungseinrichtung Messdaten einer Messdatenerfassungseinrichtung 23 nutzen, sowie Korrekturdaten oder Änderungsdaten, die nach der ersten Planungsdatenerstellung von einem Operateur mittels der zweiten Eingabeeinrichtung 24 eingegeben werden können. Die Messdateneinrichtung 23 kann beispielsweise Sensoren 23b wie Farbsensoren, eine Kamera 23a zur objektiven Erfassung der Haut-oder Haarfarbe des Patienten oder eine auf die Iris des zu behandelnden Auges gerichtete Kamera zur Erfassung der Irisfarbe umfassen oder mit derartigen Sensoren verbunden sein. Die Messdateneinrichtung kann sich für Messungen auch der Kamera 6 bedienen und diese beispielsweise auf die Iris richten oder durch diese Kamera andere Daten aufnehmen als die Bilddaten der Netzhaut. Diese Datenerfassung kann beispielsweise direkt durch die Messdatenerfassungseinrichtung 23 gesteuert oder auch durch die Bildverarbeitungseinrichtung 19 vermittelt werden.

Die Verarbeitungseinrichtung kann Planungsdaten mittels der Bildverarbeitungseinrichtung 19 oder direkt an die Anzeigeeinrichtung / das Anzeigemodul 17 senden, wo die Planungsdaten gemeinsam mit aktuellen Bildern der Netzhaut an einem Bildschirm 18 angezeigt werden können. Die Planungsdaten können dabei teilweise numerisch angezeigt werden, jedoch können sie zumindest teilweise auch unmittelbar grafisch in einem Bild der Netzhaut dargestellt werden.

Nach der Laserbehandlung kann mittels einer Erfolgskontrolleinrichtung/ Monitoringeinrichtung 25, die unter anderem von der Kamera 6 aktuelle Aufnahmen der Netzhaut 16 erhalten kann, der Erfolg der Behandlung beurteilt und an die Verarbeitungseinrichtung 20 gemeldet werden. Die Verarbeitungseinrichtung 20 weist einen Lernalgorithmus beziehungsweise eine selbstlernende Einrichtung auf, die den Zuordnungsalgorithmus zur Zuordnung von Planungsdaten zu Ausgangsdaten unter Verwendung von Trainingsdaten verbessert.

Gemäß dem Stand der Technik wurde bisher die Art und Intensität der Laserbehandlung, das heißt die Stärke und/oder Dauer sowie die zeitliche und örtliche Verteilung der Laserpulse nach Beurteilung einer Aufnahme der Netzhaut von einem Operateur nach eigener Einschätzung durchgeführt. Dabei wurden oft zunächst Titrationspulse auf die Netzhaut gerichtet und deren Wirkung beurteilt, um die Laserstärke zu skalieren.

Durch die erfindungsgemäße, weitgehend selbständige, automatisierte Zuordnung von Planungsdaten zu Bestandsdaten und Bilddaten und gegebenenfalls weiteren Daten ist es ermöglicht, jedem Zielbereich auf der Netzhaut gegebenenfalls auch ohne Titrationsschüsse Planungsdaten in objektivierter, transparenter und nachvollziehbarer Weise zuzuordnen, wobei die Planungsdaten verschiedene Kategorien von Daten, wie Laserparameter, Behandlungsorte oder -bereiche, von der Behandlung ausgenommene Orte oder Bereiche und Behandlungsmuster umfassen können.

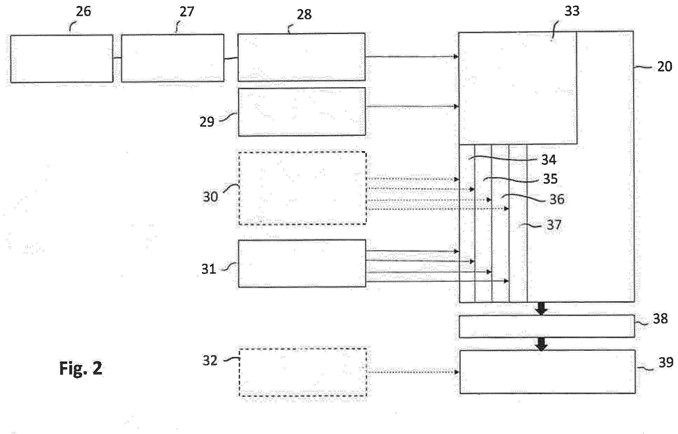

Figur 2 zeigt ein erfindungsgemäßes System mit einer Bilderfassungseinrichtung 26 der Netzhaut und einer Bildverarbeitungseinrichtung 27, die strukturierte Bilddaten 28 an die Verarbeitungseinrichtung 20 liefert. Die strukturierten Daten können beispielsweise erkannte Objekte wie Nervenknoten, Blutgefäße sowie erkannte kranke Gewebebereiche sowie Daten über die Farbe und Farbintensität der Retina sowie andere grafisch erkennbare Eigenschaften, wie Kontraste, Helligkeits-und Farbverteilungen und weitere vergleichbare Daten, enthalten. Mittels Farbintensitäts-und Helligkeitswerten lässt sich beispielsweise unter anderem die Pigmentierungsintensität der Netzhaut bestimmen, die maßgeblich für die Absorption von Laserlicht bestimmter Wellenlängen und damit maßgeblich für die notwendige Laserintensität für eine Behandlung sein kann. Unter strukturierten grafischen Daten sollen im Zusammenhang der vorliegenden Erfindung alle Aufbereitungen von reinen Pixeldaten und von diesen abgeleitete Daten verstanden werden.

Mit 29 sind in der Figur Bestandsdaten des jeweiligen Patienten bezeichnet, die der Verarbeitungseinrichtung 20 bereitgestellt werden. Dies kann durch elektronische Übertragung von Daten oder durch manuelle Eingabe erfolgen. Potenziell können der Verarbeitungseinrichtung 20 auch Messdaten einer Sensorik oder Messdatenerfassungseinrichtung zugeleitet werden, was in Figur 2 mit 30 bezeichnet ist. Diese Daten können netzhautortsabhängige Daten wie beispielsweise eine aktuell gemessene Temperaturverteilung auf der Netzhaut, oder aus OCT (Optische Kohärenztomografie) gewonnene Daten sein, die beispielsweise Ödeme anzeigen können, oder Farb- und/oder Helligkeitsmessungen der Retina. Sie können jedoch auch andere, von einem Ort der Netzhaut unabhängige Mess-oder Schätzgrößen sein, wie die Haut-, Haar- oder Irisfarbe des Patienten, die jeweils gemessen oder geschätzt und manuell eingegeben werden kann.

Die Verarbeitungseinrichtung 20 enthält ein Element 33 in Form einer Zuordnungseinrichtung, die Muster und Regeln für die Zuordnung von Planungsdaten zu Eingangsdaten (aktuellen Bilddaten beziehungsweise strukturierten Bilddaten, Bestandsdaten, sensorisch erfassten Daten) enthält.

Die Zuordnungseinrichtung gibt in dem gezeigten Beispiel Planungsdaten der Kategorien Laserparameter 34, Ortsdaten 35 für die Behandlung, Behandlungsmuster (Patterns) 36 für die Behandlung sowie Daten der für die Behandlung ausgeschlossenen Orte 37 aus.

Nach der Ausgabe und gegebenenfalls grafischen Darstellung der Planungsdaten kann durch das Element 31 mittels einer Eingabeeinrichtung ein Operateur die Planungsdaten einer, einiger oder aller Kategorien manuell ändern. Die geänderten Planungsdaten werden dann üblicherweise der Laserbehandlung zugrunde gelegt. Die geänderten Planungsdaten bilden dann endgültig die Planungsdaten 38 für die Laserbehandlung, die auch in der grafischen Darstellung 39 angezeigt werden können, beispielsweise gemeinsam mit aktuellen Bildern der Netzhaut. Das in Figur 2 dargestellte System kann so in statischer Weise funktionieren, wenn die in der Verarbeitungseinrichtung 20 gespeicherten Muster und Regeln der Zuordnungseinrichtung 33 ausreichend gut und gültig sind. Derartige Muster und Regeln können beispielsweise im Rahmen eines Expertensystems zur Verfügung gestellt und auch regelmäßig erneuert werden.

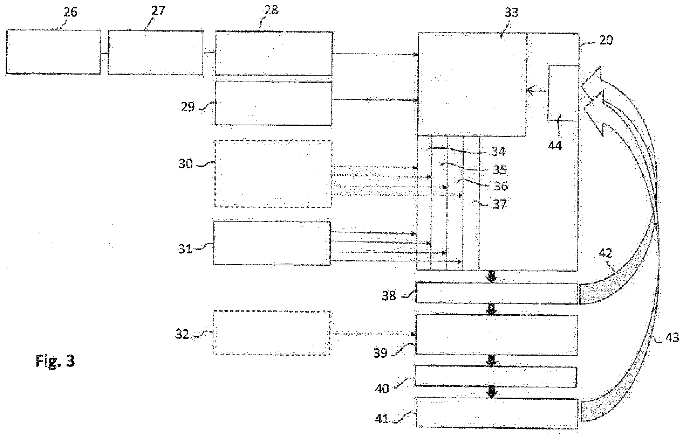

In Figur 3 ist ein System dargestellt, bei dem in der Verarbeitungseinrichtung 20 im Rahmen eines lernenden Algorithmus die Muster und Regeln, nach denen die Zuordnungsvorrichtung 33 die Zuordnung oder Erzeugung von Planungsdaten vornimmt, laufend korrigiert und/oder verbessert werden.

Dazu werden beispielsweise die Planungsdaten für die Behandlung, die sich nach manuellen Korrekturen 31 der ursprünglich durch das System geplanten Planungsdaten ergeben, im Rahmen einer Feedback-Schleife 42 an eine lernende Einrichtung 44 zurückgespielt, die die Änderungen der ursprünglichen Planungsdaten analysiert und auch feststellen kann, ob diese oder ähnliche Korrektureingaben häufiger oder nur einmalig oder selten vorkommen. Unter Berücksichtigung bestimmter Lernvorgaben, wie beispielsweise die Gewichtung bestimmter Korrekturen nach bestimmten Kriterien (Häufigkeit, Quellen für bestimmte Korrekturen, Diversität von Korrekturquellen für ähnliche Korrekturen usw.) ändert die Lerneinrichtung die abgespeicherten Muster und Regeln zumindest tendenziell, gegebenenfalls wiederholt so lange, bis in einem gegebenen Szenario keine manuellen Korrekturen mehr auftreten oder beispielsweise Korrekturen statistisch gleich häufig in verschiedene Richtungen weisen.

Gemäß den jeweils korrigierten Planungsdaten 38 und deren grafischer Darstellung 39 findet jeweils an dem Patienten eine Gewebebehandlung 40 der Retina statt.

Nach der Behandlung 40 findet eine Erfolgskontrolle im Rahmen eines Monitoring 41 beispielsweise durch oder mit Hilfe einer Monitoringeinrichtung statt. Diese Erfolgskontrolle kann eine erneute Aufnahme der Retina, beispielsweise in der Form von neu aufgenommenen Bilddaten, umfassen, jedoch grundsätzlich jede Art der Datenerhebung durch den Operateur und/oder den Patienten. Beispielsweise kann der Operateur und/oder der Patient Standardfragen beantworten, und die Antworten können in Form von Daten eingegeben und an die Verarbeitungseinrichtung als Implementierung der Feedback-Schleife 43 weitergegeben werden.

Die durch das Monitoring ermittelten Daten ermöglichen für den Einzelfall eine finale Erfolgskontrolle und können ebenfalls zur Verbesserung des Zuordnungsalgorithmus beziehungsweise der Muster und Regeln der Zuordnungseinrichtung 33 im Rahmen eines durch die Lerneinrichtung 44 gesteuerten Lernprozesses genutzt werden.

Die Verarbeitungseinrichtung 20 kann lokal am Ort der Laserbehandlung vorhanden oder auch als zentrale Einrichtung ausgebildet sein, die von den übrigen in den Figuren 2 und 3 gezeigten Elementen entfernt lokalisiert ist und die Datenverarbeitung und Ermittlung von Planungsdatensätzen für eine Mehrzahl von Behandlungsstationen mit Behandlungslasern vornimmt. Im Zuge der Zusammenarbeit mit einer großen Zahl von Behandlungsstationen erhält die Verarbeitungseinrichtung, wenn sie als lernende Einrichtung aufgebaut ist, eine große Anzahl an Trainingsdaten, so dass der Zuordnungsalgorithmus für die Planungsdaten zügig verbessert werden kann.

Die Eingabe oder Übertragung von Bestandsdaten kann grundsätzlich am Ort der Laserbehandlung oder auch nichtlokal, also beispielsweise in einer Zentrale oder auch in einem verteilten Rechnersystem, erfolgen. Ebenso können Korrektureingaben eines Operateurs oder eines anderen Experten, ebenso wie das Monitoring, wenn es ohne die direkte Erhebung von Messdaten der Retina erfolgt, vor Ort an der Behandlungseinrichtung in unmittelbarer Nähe des Patienten oder davon entfernt in einer Zentrale oder über ein verteiltes Rechnersystem erfolgen.

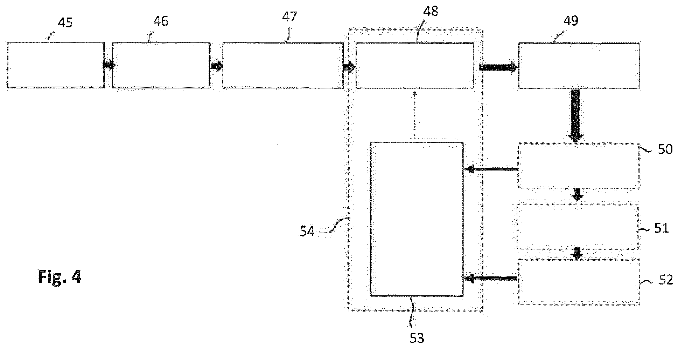

Figur 4 zeigt einen beispielhaften Ablaufplan für ein Verfahren gemäß der Erfindung zur Erstellung einer Planungsdatei für eine Laserbehandlung der Netzhaut eines Auges.

In einem ersten Schritt 45 werden alle vorbekannten Daten, also die Bestandsdaten des Patienten, wie beispielsweise eine Diagnose, eine Behandlungsindikation sowie gegebenenfalls ein Behandlungsstatus, falls bereits Vorbehandlungen stattgefunden haben, und andere bekannte physiologische Daten abgerufen und in das System importiert. Dies kann unter anderem durch Datentransport in elektronischer Form oder auch durch manuelle Eingabe erfolgen. In einem zweiten Schritt 46 wird mittels einer Kamera oder einer anderen bildgebenden Einrichtung eine aktuelle Aufnahme der Netzhaut des Patienten erstellt, das heißt eine Datenrepräsentation von ortsabhängigen Daten der Netzhaut. Diese Daten werden in einem dritten Schritt 42 derart verarbeitet, dass Strukturen und/oder Objekte erkannt werden, und die Daten werden in strukturierte Daten umgewandelt. Im nächsten, vierten Schritt 48 wird unter Berücksichtigung der Bestandsdaten und der strukturierten Bilddaten ein Planungsdatensatz für eine Netzhautbehandlung erstellt, wobei auf bestehende, gespeicherte Vorlagen in Form von Mustern und Regeln zurückgegriffen wird. Die Planungsdaten können auch bereits eine Vorlage für einen Bericht über die Vorgaben und die Durchführung und gegebenenfalls den Erfolg der Behandlung enthalten. Die Planungsdaten werden dann gegebenenfalls in einem folgenden Schritt 49 durch einen Operateur korrigiert. Die korrigierten Planungsdaten können in einer Ausführungsform des Verfahrens in einem Schritt 50 in einen Trainingsprozess 54 geschickt werden, der den Vorgang 48 zur Erzeugung von Planungsdaten nach den bestehenden Mustern und Regeln beeinflusst und dazu innerhalb des Trainingsmanagements 53 Erfahrungsdaten sammelt, bewertet und gegebenenfalls als Trainingsdaten freigibt und auch gewichtet.

Nach einer in Figur 4 selbst nicht dargestellten Behandlung der Netzhaut gemäß den geltenden Planungsdaten wird ein Monitoring 51 in Form einer Erfolgsbewertung oder Erfolgserfassung durchgeführt, die auf der Basis von Messdaten und/oder der Einschätzung einer Person automatisiert oder manuell erfolgen kann. In einem weiteren Schritt 52 kann die Erfolgsbewertung in den Trainingsprozess 54 eingebracht werden, um den Erzeugungsprozess 48 von Planungsdaten zu verbessern.

Durch den erfindungsgemäßen Prozess kann das Verfahren zur Erzeugung von Planungsdateien/Planungsdaten für die Laserbehandlung der Netzhaut laufend verbessert werden.

Durch Verwendung eines auf diese Weise verbesserten automatisierten Systems können die verschiedenen bekannten Behandlungen in zuverlässiger Weise nachvollziehbar und objektiviert mit geringem Aufwand geplant werden.

## Patentansprüche

1. System zur Erstellung von Planungsdatensätzen (34, 35, 36, 37) für die Gewebebehandlung der Netzhaut (16) des Auges (4) eines Patienten mittels eines Lasers (2),
- mit einer Kamera (6,7), die dazu eingerichtet ist, laufend Aufnahmen der Netzhaut zu erfassen,
- mit einer Bildverarbeitungseinrichtung (19), die dazu eingerichtet ist, aus durch die Kamera erfassten Aufnahmen aktuelle strukturierte Bilddaten der Netzhaut zu erzeugen und bereitzustellen,
- mit einer Bestandsdatenerfassungseinrichtung (21,22), die dazu eingerichtet ist, Bestandsdaten eines Patienten zu erfassen und/oder bereitzustellen, und
- mit einer Verarbeitungseinrichtung (20), die dazu eingerichtet ist, den aktuellen strukturierten Bilddaten der Netzhaut und den Bestandsdaten des Patienten Planungsdatensätze für die Gewebebehandlung zuzuordnen, wobei in der Verarbeitungseinrichtung Vorgaben, insbesondere Muster und Regeln, für die Zuordnung von Planungsdaten zu Bilddaten und Bestandsdaten von Patienten gespeichert sind.

2. System nach Anspruch 1 mit einer Änderungseinrichtung (24), die die manuelle Änderung eines Planungsdatensatzes ermöglicht.

3. System nach Anspruch 1 oder 2, **dadurch gekennzeichnet, dass** die Bestandsdatenerfassungseinrichtung (21, 22) dazu eingerichtet ist, Diagnosedaten und/oder Indikationsdaten und/oder Daten einer durchgeführten Vorbehandlung und/oder vorbekannte physiologische Daten des Patienten und/oder bereits vorhandene, außerhalb des Systems gespeicherte Aufnahmen der Netzhaut (16) des Patienten zu erfassen und bereitzustellen.

4. System nach Anspruch 1, 2 oder 3, **dadurch gekennzeichnet, dass** eine Messdatenerfassungseinrichtung (23) vorgesehen ist, die eine oder mehrere Sensoreinrichtungen zur Erfassung und Bereitstellung von physiologischen Daten des Patienten, insbesondere Daten, die vom jeweiligen Ort auf der Netzhaut abhängen, und/oder Daten, die nicht von einem Ort auf der Netzhaut abhängen, aufweist, und dass die Verarbeitungseinrichtung dazu eingerichtet ist, bei der Zuordnung von Planungsdaten die erfassten physiologischen Daten des Patienten zu berücksichtigen.

5. System nach einem der Ansprüche 1, 2, 3 oder 4, **gekennzeichnet durch** ein Anzeigemodul (17, 18), das die grafische Darstellung eines Planungsdatensatzes innerhalb einer durch die Kamera (6, 7) aktuell erfassten Aufnahme der Netzhaut (16) bewirkt.

6. System nach Anspruch 5, **gekennzeichnet durch** eine Trackingeinrichtung, die bei einer Verschiebung der aktuell durch die Kamera (6, 7) erfassten Aufnahme eine Nachführung der Darstellung des Planungsdatensatzes bewirkt.

7. System nach einem der Ansprüche 1 bis 6, **dadurch gekennzeichnet, dass** die Verarbeitungseinrichtung (20) eine Speichereinrichtung (33) aufweist, die zur Speicherung von Bestandsdaten von Patienten und von diesen zugeordneten Planungsdatensätzen eingerichtet ist, sowie insbesondere zusätzlich zur Speicherung von jeweils zu den vorgenannten Daten zugehörigen, mittels einer Messdatenerfassungseinrichtung (23) erfassten physiologischen Daten und/oder zur Speicherung von jeweils vorgenommenen manuellen Änderungen der Planungsdatensätze.

8. System nach einem der Ansprüche 1 bis 6, **dadurch gekennzeichnet, dass** die Verarbeitungseinrichtung (20) eine Speichereinrichtung (33) aufweist, die zur Speicherung von Bestandsdaten von Patienten sowie von mit dem System erzeugten strukturierten Bilddaten und/oder Aufnahmen der Netzhaut (16) des Patienten und von diesen zugeordneten Planungsdatensätzen (34, 35, 36, 37) eingerichtet ist, sowie insbesondere zusätzlich zur Speicherung von jeweils zu den vorgenannten Daten zugehörigen, mittels einer Messdatenerfassungseinrichtung (23) erfassten physiologischen Daten und/oder zur Speicherung von jeweils vorgenommenen manuellen Änderungen der Planungsdatensätze.

9. System nach einem der Ansprüche 1 bis 8, **gekennzeichnet durch** eine Überprüfungseinrichtung (25), die nach Durchführung einer Behandlung der Netzhaut (16) Daten zu den Auswirkungen der Behandlung erfasst und bereitstellt.

10. System nach einem der Ansprüche 1 bis 8, **dadurch gekennzeichnet, dass** die Verarbeitungseinrichtung (20) als selbstlernende Einrichtung ausgebildet ist, in der die Zuordnungsfunktion, die den Bestandsdaten und insbesondere den strukturierten Bilddaten sowie insbesondere gemessenen physiologischen Daten eines Patienten Planungsdaten (34, 35, 36, 37), insbesondere unter anderem Zielwerte zu Messdaten, zuordnet, dadurch trainiert wird, dass ihr jeweils zu einem Patienten einerseits zumindest Daten von wenigstens einer der folgenden Kategorien:
- Bestandsdaten
- strukturierte Bilddaten
- gemessene physiologische Daten
und andererseits zugeordnete Planungsdaten, insbesondere unter anderem Zielwerte zu Messdaten, sowie manuelle Planungsdatenänderungen und insbesondere Daten zu einer nach Durchführung der geplanten Behandlung erfassten Auswirkung der Behandlung als Trainingsdaten vorgegeben werden.

11. System nach einem der Ansprüche 1 bis 10, **dadurch gekennzeichnet, dass** die Planungsdatensätze (34, 35, 36, 37) Planungsdaten von einer, zwei, drei, vier oder fünf der folgenden Kategorien enthalten: Laserparameter (34), Ortsdaten für die Laserbehandlung (35), Behandlungsmuster (36), Ortsdaten für Bereiche, die von der Laserbehandlung ausgeschlossen sind (37), Zielwerte für Messdaten.

12. Vorrichtung zur Erstellung von Planungsdatensätzen (34, 35, 36, 37) für die Gewebebehandlung der Netzhaut (16) eines Auges (4) mittels eines Laserstrahls (11),
- mit einer Kamera (6, 7), die dazu eingerichtet ist, laufend Aufnahmen der Netzhaut (16) zu erfassen,
- mit einer Bildverarbeitungseinrichtung (19), die aus durch die Kamera (6, 7) erfassten Aufnahmen aktuelle strukturierte Bilddaten der Netzhaut erzeugt und bereitstellt,
- mit einer Bestandsdatenerfassungseinrichtung (21, 22), die dazu eingerichtet ist, Bestandsdaten eines Patienten zu erfassen und/oder bereitzustellen,
- und mit einer Verarbeitungseinrichtung (20), die dazu eingerichtet ist, den aktuellen strukturierten Bilddaten der Netzhaut und den Bestandsdaten des Patienten Planungsdatensätze (34, 35, 36, 37) zuzuordnen, wobei in der Verarbeitungseinrichtung Vorgaben, insbesondere Muster und Regeln, für die Zuordnung von Planungsdaten zu strukturierten Bilddaten und Bestandsdaten von Patienten gespeichert sind.
- und mit einer Änderungseinrichtung (22), die die manuelle Änderung eines Planungsdatensatzes ermöglicht.

13. Vorrichtung nach Anspruch 12 **gekennzeichnet durch** eine Änderungseinrichtung (22), die die manuelle Änderung eines Planungsdatensatzes ermöglicht.

14. Verfahren zur Erstellung von Planungsdatensätzen (34, 35, 36, 37) für die Gewebebehandlung der Netzhaut (16) eines Auges (4) eines Patienten mittels eines Laserstrahls (11),
- bei dem mit einer Kamera (6, 7) wenigstens eine Aufnahme der Netzhaut erfasst wird,
- bei dem mit einer Bildverarbeitungseinrichtung (19) aus durch die Kamera erfassten Aufnahmen aktuelle strukturierte Bilddaten der Netzhaut erzeugt und bereitgestellt werden,
- bei dem im Rahmen einer Bestandsdatenerfassungseinrichtung (21, 22) Bestandsdaten eines Patienten erfasst und/oder bereitgestellt werden,
- und bei dem durch eine Verarbeitungseinrichtung (20) den aktuellen strukturierten Bilddaten der Netzhaut und den vorbekannten Bestandsdaten des Patienten aufgrund von Vorgaben, insbesondere Mustern und Regeln, die in der Verarbeitungseinrichtung (20) gespeichert sind, Planungsdatensätze zugeordnet werden.

15. Verfahren nach Anspruch 14, **dadurch gekennzeichnet, dass** zugeordnete Planungsdatensätze (34, 35, 36, 37) nach der Zuordnung manuell geändert werden.

16. Verfahren nach Anspruch 14 oder 15, **dadurch gekennzeichnet, dass** physiologische Daten des Patienten insbesondere mit einer Messdatenerfassungseinrichtung (23) erfasst und bereitgestellt sowie bei der Zuordnung von Planungsdatensätzen (34, 35, 36, 37) durch die Verarbeitungseinrichtung (20) berücksichtigt werden.

17. Verfahren nach einem der Ansprüche 14 bis 16, **dadurch gekennzeichnet, dass** die Verarbeitungseinrichtung (20) als selbstlernende Einrichtung ausgebildet ist und die Zuordnungsfunktion, die den Bestandsdaten und den strukturierten Bilddaten sowie insbesondere gemessenen physiologischen Daten eines Patienten Planungsdaten zuordnet, dadurch trainiert wird, dass ihr zu einer Mehrzahl von Patienten jeweils die Bestandsdaten, strukturierte Bilddaten, zugeordnete Planungsdaten (34, 35, 36, 37), Daten der manuellen Planungsdatenänderungen sowie insbesondere gemessene oder erfasste physiologische Daten und/oder Daten zu der nach Durchführung der jeweils geplanten Behandlung erfassten Auswirkung der Behandlung vorgegeben werden.

18. Verfahren nach einem der Ansprüche 14 bis 17, **dadurch gekennzeichnet, dass** als Planungsdatensätze (34, 35, 36, 37) Planungsdaten von einer, zwei, drei, vier oder fünf der folgenden Kategorien ermittelt werden: Laserparameter (34), Ortsdaten für die Laserbehandlung (35), Behandlungsmuster (35), Ortsdaten für Bereiche, die von der Laserbehandlung ausgeschlossen sind (36), Zielwerte für Messdaten.

19. Verfahren zum Betrieb eines Systems nach einem der Ansprüche 1 bis 11 und/oder einer Vorrichtung gemäß Anspruch 12 oder 13, **dadurch gekennzeichnet, dass** ein Planungsdatensatz erstellt und dann in einer separaten oder einer in das System oder die Vorrichtung integrierten Behandlungseinrichtung mittels eines Behandlungslasers eine Behandlung gemäß dem Planungsdatensatz durchgeführt wird.
